# EUROPÄISCHE PATENTSCHRIFT

(11) **EP 1 293 177 B1**
(45) Veröffentlichungstag und Bekanntmachung des Hinweises auf die Patenterteilung: **02.03.2005**
(21) Anmeldenummer: 01122285.8
(22) Anmeldetag: 18.09.2001
(51) Int. Cl.: A61F 2/06

(54) **Stent**
Stent
Stent

(43) Veröffentlichungstag der Anmeldung: 19.03.2003
(60) Teilanmeldung: 04030668.0
(73) Patentinhaber: Abbott Laboratories Vascular Enterprises Limited, Dublin 2 (IE)
(72) Erfinder: Calisse, Jorge, Dr., 10785 Berlin (DE)
(74) Vertreter: Schmitz, Hans-Werner, Dipl.-Ing.

(56) Entgegenhaltungen:
- WO-A-99/17680
- WO-A-99/38458
- WO-A-99/49928
- US-B1- 6 261 318

## Beschreibung

Die Erfindung betrifft einen Stent gemäß dem Oberbegriff des Anspruches 1. Ein derartigen Stent in aus de WO 99 49 928, bekannt.

Aus dem Stand der Technik sind unterschiedlichste Ausgestaltungen von Stents, insbesondere Koronarstents als ballonoder selbstexpandierbare Stents, vorbekannt. Der Stent bildet eine Gefäßprothese, die aus körperverträglichem Material besteht. Der Stent bzw. die Stentprothese wird dazu verwendet, Blutgefäße oder aber auch andere Körperöffnungen aufzuweiten und im aufgeweiteten Zustand zu halten. Zu diesem Zweck wird der Stent in einem nicht-expandierten Zustand, üblicherweise mit Hilfe eines Ballonkatheters, auf dem der Stent aufgecrimpt ist, im Körper des Patienten positioniert und nachfolgend expandiert. Beim Expandieren werden die einzelnen Stegbereiche des Stents verformt, so dass dieser dauerhaft in der expandierten Form bleibt.

Ein weiterer Stent der im Oberbegriff des Anspruches 1 angegebenen Art ist beispielsweise aus dem DE-U-297 08 689.8 bekannt. Bei diesem Stent sind als Verbinder zwischen den Stegmustern der Stegstruktur der Stentwand s-förmige Verbindungselemente vorgesehen. Obwohl diese Verbindungselemente im nicht-expandierten Zustand zu einer sehr flexiblen und im expandierten Zustand zu einer Stent-Konstruktion mit hoher Radialkraft bzw. Radialkraft-Aufnahmefähigkeit führen, ergeben sich jedoch Verbesserungsmöglichkeit en dahingehend, dass nicht in jedem Falle ausgeschlossen werden kann, dass sich die Verbindungselemente beim Aufweiten aus der Wandebene nach aussen oder innen herauswölben und dadurch das umgebende Gewebe etwas in Mitleidenschaft gezogen wird bzw. die Bildung von Thrombosen oder Restenosen gefördert wird.

Es ist daher Aufgabe der vorliegenden Erfindung, einen Stent der im Oberbegriff des Anspruches 1 angegebenen Art zu schaffen, der im nicht-expandierten Zustand äusserst flexibel ist und bei dem beim Aufweiten verhindert werden kann, dass sich die Verbindungselemente aus der Wandfläche des Stents herausbewegen können.

Die Lösung dieser Aufgabe erfolgt durch die Merkmale des Anspruches 1.

Mit dem erfindungsgemäßen Stent wird eine sehr flexible Struktur beim Biegen und Komprimieren erreicht. Es ist insbesondere vorteilhafterweise möglich zu verhindern, dass beim Aufweiten des Stents die Verbindungselemente das Gewebe im expandierten Zustand des Stents beeinträchtigen, und dass das Lumen des Stentinneren vermindert wird.

Der Grund für diese vorteilhafte Wirkung ist vor allem darin zu sehen, dass sich die Verbindungselemente sowohl beim Biegen (wie zum Beispiel beim Platzieren des Stents in einem gekrümmten Gefäß) und beim Ausdehnen (wie zum Beispiel beim Aufweiten mittels des Ballons) verkürzen, was durch die besondere Anordnung der Verbindungsstege der Verbindungselemente mit den vorgesehenen Scharnieren bzw. Gelenken erreicht wird.

Die Unteransprüche haben vorteilhafte Weiterbildungen der Erfindung zum Inhalt.

Die Verbindungsstege der Verbindungselemente sind im nicht-expandierten oder komprimierten Zustand der Stegstruktur zumindest annähernd vorzugsweise exakt im rechten Winkel zueinander angeordnet.

Hierbei ist es möglich, die Breite der Verbindungsstege jeweils gleich zu machen oder, um eine erhöhte Flexibilität zu erreichen, die Breite des mittleren Verbindungssteges etwas geringer auszuführen als die Breite der an den mittleren Steg anschließenden Verbindungsstege.

Die Verbindungsstege, die mit den benachbarten Stegen der Stegstruktur verbunden sind, werden bei einer besonders bevorzugten Ausführungsform ebenfalls über Scharniere angebracht.

Als besonders bevorzugte und besonders einfache Ausführungsform für die Scharniere sind Filmscharniere vorgesehen.

Bevorzugterweise besteht der Stent aus einem biokompatiblen Material, das bei einer besonders bevorzugten Ausführungsform eine Nickel-Titan-Legierung oder Edelstahl darstellt.

Weitere Einzelheiten, Merkmale und Vorteile der Erfindung ergeben sich aus nachfolgender Beschreibung anhand der Zeichnung.

Es zeigt:
- Fig. 1: eine schematisch stark vereinfachte Prinzipdarstellung der Grundform des erfindungsgemäßen Stents,
- Fig. 2: eine Darstellung eines Teiles der Stegstruktur der Wand des Stents im nicht-expandierten Zustand,
- Fig. 3: eine Prinzipdarstellung der erfindungsgemäßen Verbindungselemente zur Erläuterung ihrer Funktionsweise, und
- Fig. 4A, B: jeweils eine der Fig. 2 entsprechende Darstellung der Anordnung des erfindungsgemäßen Stents in einem gekrümmten Gefäß.

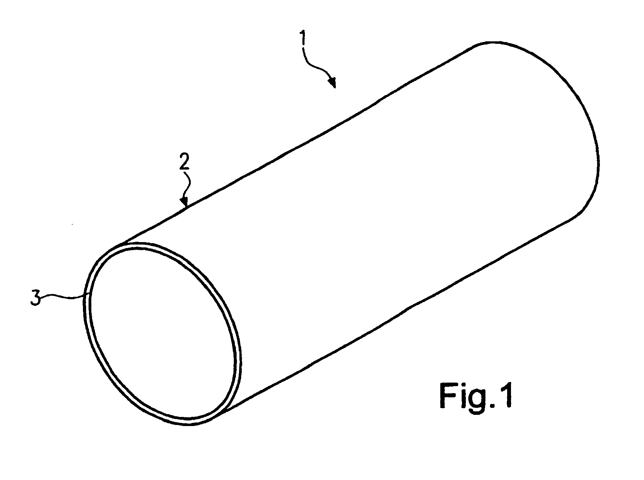

Fig. 1 zeigt den grundsätzlichen Aufbau eines erfindungsgemäßen Stents 1, der einen flexiblen, rohrförmigen Körper 2 mit einer Wand 3 aufweist, von der in Fig. 1 die Stirnansicht dargestellt ist. Der Stent gemäß der vorliegenden Erfindung kann als ballonexpandierbarer oder selbstexpandierbarer Stent ausgebildet sein.

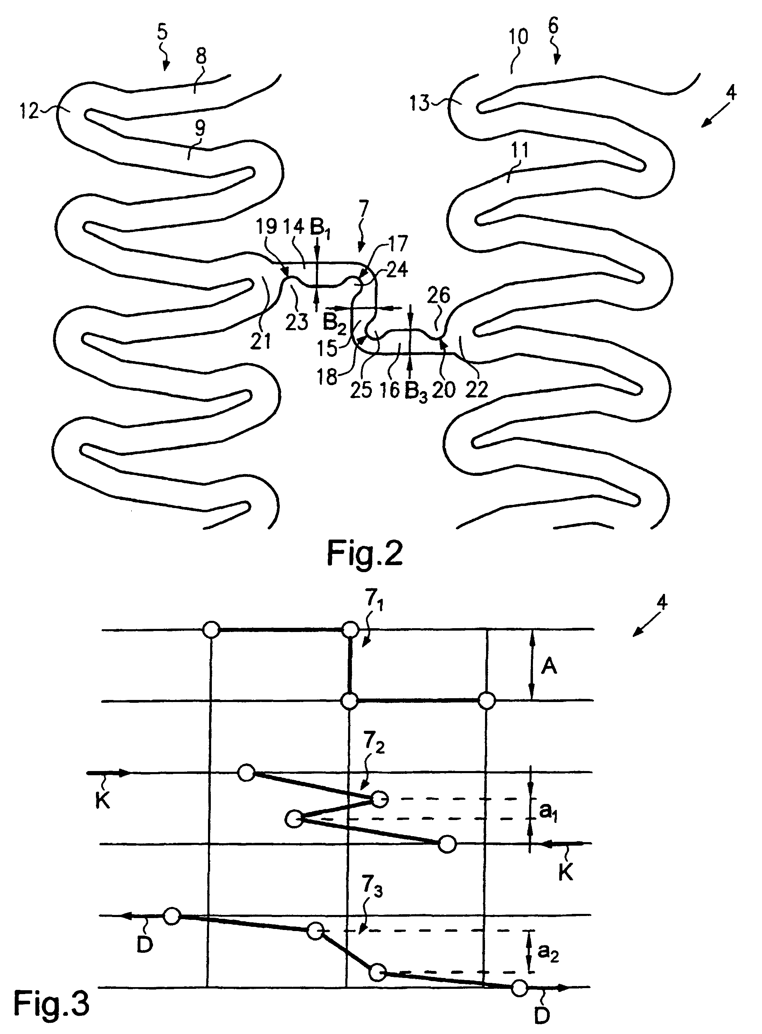

In Fig. 2 ist der Aufbau der Stegstruktur des erfindungsgemäßen Stents gezeigt.

Die Wand 3 des Körpers 2 des Stents 1 weist eine Stegstruktur 4 auf, die von einem nicht-expandierten Zustand in einen expandierten Zustand überführbar ist.

Hierbei weist die Stegstruktur 4 eine Vielzahl von benachbarten Stegmustern auf, von denen in Fig. 2 beispielhaft die Stegmuster 5 und 6 dargestellt sind. Die Stegmuster 5 und 6 sind untereinander durch zumindestens ein Verbindungselement 7 miteinander verbunden.

Zur Verdeutlichung des Aufbaus der Stegmuster 5 und 6 sind beispielhaft die Stege 8 und 9 sowie der diese Stege 8 und 9 verbindende Bogen 12 des Stegmusters 5 bzw. die entsprechenden Teile 10, 11 und 13 des Stegmusters 6 mit Bezugsziffern identifiziert.

Das Verbindungselement 7 weist drei im Winkel zueinander angeordnete Verbindungsstege 14, 15, 16 auf. Die Verbindungsstege 14 und 15 sind hierbei über ein Scharnier 17 und die Verbindungsstege 15 und 16 über ein Scharnier 18 miteinander verbunden. Der äussere Verbindungssteg 15 ist ferner über ein Scharnier 19 mit einem Bogen 21 und der zweite äussere Verbindungssteg 16 über ein Scharnier 20 mit dem Bogen 22 entsprechend der Stegstrukturen 5 bzw. 6 verbunden.

Bei der in Fig. 2 besonders bevorzugten Ausführungsform schließen die Verbindungsstege 14, 15 und 16 jeweils rechte Winkel zwischeneinander ein. Die Scharniere 17, 18, 19 und 20 sind als Filmscharniere ausgebildet, die durch das Vorsehen von annähernd halbkreisförmigen Ausmündungen 23, 24, 25 bzw. 26 in den jeweiligen Verbindungsstegen gebildet werden.

Ferner ist bei der in Fig. 2 dargestellten besonders bevorzugten Ausführungsform die Breite B1 gleich der Breite B2 und diese wiederum gleich der Breite B3 der Verbindungsstege 14, 15 bzw. 16. Zur Erhöhung der Flexibilität ist es jedoch möglich, insbesondere die Breite B2 des Verbindungssteges 15 im Vergleich zur Breite B1 und zur Breite B3 geringer auszubilden.

In Fig. 3 ist die Wirkungsweise des erfindungsgemäßen Verbindungselementes 7 innerhalb der Stegstruktur 4 verdeutlicht. Hierbei stellt die Anordnung 7₁ die Ausbildung des Verbindungselementes 7 gemäß Fig. 2 in schematisch vereinfachter Darstellung dar. Es ergibt sich ein Abstand A der der Länge des mittleren Verbindungssteges 15 entspricht.

Der Zustand 7₂ des Verbindungselementes 7 verdeutlicht die Stellung der Stege 14, 15 und 16 in einem komprimierten Zustand, der durch die beiden Pfeile K symbolisiert wird. Hierbei ergibt sich ein Abstand a1 zwischen den Scharnieren 17 und 18, der kleiner dem Abstand A ist.

Auch bei dem Zustand 7₃, der einem expandierten Zustand entspricht, ergibt sich ein Abstand a₂, der ebenfalls kleiner ist als der Abstand A.

Dies bedeutet, dass sich eine Verkürzung des Verbindungselementes 7 sowohl im komprimierten wie auch im expandierten Zustand ergibt, der es verhindert, dass die Verbindungselemente 7 aus der Wandebene der Stegstruktur 4 heraus vortreten, so dass insbesondere im implantierten Zustand eine Verletzung des umgebenden Gewebes des jeweiligen Volumens vermieden werden kann. Ferner ergibt sich der Vorteil, dass die Verbindungselemente in gekrümmten Gefässen eine gleichmäßige Wandabdeckung durch die Stegmuster und Flexibilität der gesamten Stentkonstruktion ergeben.

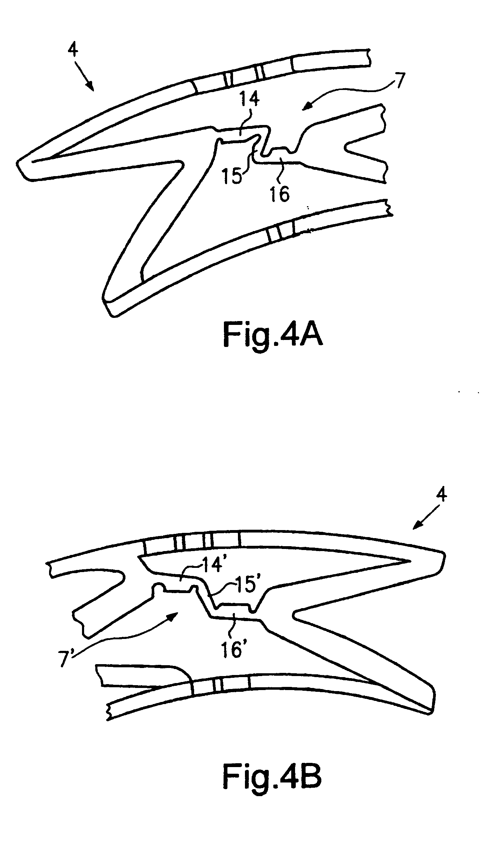

Hierzu ist auf die Figur 4A und 4B zu verweisen, in der ein Ausschnitt der Stegstruktur 4 jeweils bei einer Anordnung in einem gekrümmten Gefäß dargestellt ist.

Figur 4A zeigt hierbei den gestauchten Zustand des Verbindungselementes 7 mit seinen Stegen 14 bis 16, während 4B den gestreckten Zustand eines entsprechenden Verbindungselementes 7' mit seinen Stegen 14' bis 16' illustriert und verdeutlicht.

## Patentansprüche

1. Stent (1)
- mit einem rohrförmigen flexiblen Körper (2), dessen Wand (3) eine Stegstruktur (4) aufweist, die von einem nicht-expandierten Zustand in einen expandierten Zustand überführbar ist;
- wobei die Stegstruktur (4) eine Vielzahl von benachbarten Stegmustern (5,6) aufweist, die sich aneinander anreihende Stege (8,9 bzw. 10,11) aufweisen; und
- wobei die Stegmuster (5,6) durch zumindestens ein Verbindungselement (7) miteinander verbunden sind, **dadurch gekennzeichnet**
- **dass** das Verbindungselement (7) drei winklig zueinander angeordnete Verbindungsstege (14,15,16) aufweist, die über Scharniere (17,18) miteinander verbunden sind.

2. Stent nach Anspruch 1, **dadurch gekennzeichnet, dass** die Verbindungsstege (14-16) im nicht-expandierten oder komprimierten Zustand der Stegstruktur (4) zumindestens annähernd im rechten Winkel zueinander angeordnet sind.

3. Stent nach Anspruch 1 oder 2, **dadurch gekennzeichnet, dass** die Verbindungsstege (14-16) alle die gleiche Breite (B₁,B₂,B₃) aufweisen.

4. Stent nach Anspruch 1 oder 2, **dadurch gekennzeichnet, dass** die Verbindungsstege (14, 15, 16) unterschiedliche Breite aufweisen.

5. Stent nach Anspruch 1 oder 2, **dadurch gekennzeichnet, dass** die Breite (B₂) des mittleren Verbindungssteges (15) geringer als die Breite (B₁ bzw. B₃) der äusseren Verbindungsstege (14,16) ist.

6. Stent nach einem der Ansprüche 1 bis 5, **dadurch gekennzeichnet, dass** die äusseren Verbindungsstege (14,16) über je ein Scharnier (19,20) mit dem benachbarten Steg (21,22) der Wandstruktur (4) verbunden sind.

7. Stent nach einem der Ansprüche 1 bis 6, **dadurch gekennzeichnet, dass** die Scharniere (17-20) als Filmscharniere ausgebildet sind.

8. Stent nach einem der Ansprüche 1 bis 7, **dadurch gekennzeichnet, dass** das Material der Wand (3) des Körpers (2) aus körperverträglichem Material besteht.

9. Stent nach Anspruch 8, **dadurch gekennzeichnet, dass** die Stegstruktur (4) und die Verbindungselemente (7) aus einer Nickel-Titan-Legierung, aus Kunststoff oder nicht rostendem Stahl bestehen.

10. Stent nach einem der Ansprüche 1 bis 9, **dadurch gekennzeichnet, dass** die Länge der Verbindungsstege (14-16) gleich oder unterschiedlich ist.

## Claims

1. Stent (1)
- with a tubular flexible body (2), whose wall (3) exhibits a strut structure (4), which can be brought from a non-expanded state into an expanded state;
- whereby said strut structure (4) exhibits a multitude of adjacent strut patterns (5, 6), which exhibit joined-up struts arranged side by side (8, 9 and 10, 11 resp.); and
- whereby said strut patterns (5, 6) are linked together by at least one connecting element 7,
- **characterised in that** said connecting element (7) exhibits three connecting struts (14, 15 and 16) arranged at an angle to one another, which are linked together via hinges (17, 18).

2. Stent according to claim 1, **characterised in that**, in the non-expanded or compressed state of said strut structure (4), said connecting struts (14-16) are arranged at least roughly at a right angle to one another.

3. Stent according to one of claims 1 and 2, **characterised in that** said connecting struts (14-16) all exhibit the identical width (B₁, B₂, B₃).

4. Stent according to one of claims 1 and 2, **characterised in that** said connecting struts (14, 15, 16) exhibit differing widths.

5. Stent according to one of claims 1 and 2, **characterised in that** said width (B₁₎ of the middle connecting strut is smaller than said width (B₁ or B₃) of said outer connecting strut (14, 16).

6. Stent according to one of claims 1 to 5, **characterised in that** said outer connecting struts (14, 16) are each linked via a hinge (19, 20) to the adjacent strut (21, 22) of said wall structure (4).

7. Stent according to one of claims 1 to 6, **characterised in that** said hinges (17-20) are formed as film hinges.

8. Stent according to one of claims 1 to 7, **characterised in that** said wall (3) of said body (2) consists of bio-compatible material.

9. Stent according to claim 8, **characterised in that** said strut structure (4) and said connecting elements (7) consist of a nickel-titanium alloy, plastic or stainless steel.

10. Stent according to one of claims 1 to 9, **characterised in that** the length of said connecting struts (14-16) is the same or differs.

## Revendications

1. Endoprothèse coronaire (1)
- avec un corps (2) tubulaire flexible, dont la paroi (3) comporte une structure à barrettes (4) qui peut passer d'une position non expansée à une position expansée ;
- la structure à barrettes (4) comportant une pluralité de modèles voisins de barrettes (5, 6) qui comportent des barrettes (8, 9, respectivement 10, 11) alignées entre elles ; et
- les modèles de barrettes (5, 6) étant assemblés entre eux par au moins un élément de liaison (7), **caractérisée en ce que**
- l'élément de liaison (7) comporte trois barrettes de liaison (14, 15, 16) agencées en angle les unes par rapport aux autres et assemblées entre elles par des charnières (17, 18).

2. Endoprothèse coronaire selon la revendication 1, **caractérisée en ce que** les barrettes de liaison (14-16), dans la position non expansée ou comprimée de la structure à barrettes (4), sont agencées les unes par rapport aux autres au moins approximativement à angle droit.

3. Endoprothèse coronaire selon la revendication 1 ou 2, **caractérisée en ce que** les barrettes de liaison (14-16) ont toutes la même largeur (B₁, B₂, B₃).

4. Endoprothèse coronaire selon la revendication 1 ou 2, **caractérisée en ce que** les barrettes de liaison (14, 15, 16) ont des largeurs différentes.

5. Endoprothèse coronaire selon la revendication 1 ou 2, **caractérisée en ce que** la largeur (B₂) de la barrette de liaison centrale (15) est inférieure à la largeur (B, et B₃) des barrettes de liaison (14, 16) extérieures.

6. Endoprothèse coronaire selon l'une des revendications 1 à 5, **caractérisée en ce que** les barrettes de liaison (14, 16) extérieures sont assemblées chacune par l'intermédiaire d'une charnière (19, 20) avec la barrette (21, 22) voisine de la structure de paroi (4).

7. Endoprothèse coronaire selon l'une des revendications 1 à 6, **caractérisée en ce que** les charnières (17-20) sont conçues sous forme de films formant charnière.

8. Endoprothèse coronaire selon l'une des revendications 1 à 7, **caractérisée en ce que** la matière de la paroi (3) du corps (2) est une matière biocompatible.

9. Endoprothèse coronaire selon la revendication 8, **caractérisée en ce que** la structure à barrettes (4) et les éléments de liaison (7) sont réalisés dans un alliage de nickel-titane, en matière plastique ou en acier inoxydable.

10. Endoprothèse coronaire selon l'une des revendications 1 à 9, **caractérisée en ce que** les barrettes de liaison (14-16) ont la même longueur ou une longueur différente.
